Europäisches Patentamt

European Patent Office

Office européen des brevets

(11) Veröffentlichungsnummer: **0 235 651**
**B1**

(12) **EUROPÄISCHE PATENTSCHRIFT**

(45) Veröffentlichungstag der Patentschrift: **24.10.90**

(51) Int. Cl.⁵: **C 07 D 295/02**

(21) Anmeldenummer: **87102116.8**

(22) Anmeldetag: **14.02.87**

(54) Verfahren zur Herstellung von N-Methylpiperazin.

(30) Priorität: **18.02.86 DE 3605005**

(43) Veröffentlichungstag der Anmeldung:
**09.09.87 Patentblatt 87/37**

(45) Bekanntmachung des Hinweises auf die
Patenterteilung:
**24.10.90 Patentblatt 90/43**

(84) Benannte Vertragsstaaten:
**AT BE CH DE FR GB IT LI NL SE**

(56) Entgegenhaltungen:
**EP-A-0 137 478**
**GB-A- 902 570**
**US-A-2 525 223**

(73) Patentinhaber: **BASF Aktiengesellschaft**
**Carl-Bosch-Strasse 38**
**D-6700 Ludwigshafen (DE)**

(72) Erfinder: **Schroeder, Wolfgang, Dr.**
**51 Seebacher Strasse**
**D-6702 Bad Duerkheim (DE)**

**Die Akte enthält technische Angaben, die nach dem Eingang der Anmeldung eingereicht wurden und die nicht in dieser Patentschrift enthalten sind.**

Courier Press, Leamington Spa, England.

**Beschreibung**

Die Erfindung betrifft ein Verfahren zur Herstellung von N-Methylpiperazin.

N-Methylpiperazin ist ein wichtiges Zwischenprodukt bei der Synthese von Farbstoffen, Pharmazeutika und Pflanzenschutzmitteln.

Die bisher bekannt gewordenen Verfahren zur Herstellung von N-Methylpiperazin lassen sich in zwei Gruppen einteilen.

Eine Verfahrensart besteht in der Methylierung von Piperazin. Ein derartiges Verfahren ist beispielsweise in der DE—B—2 531 060 beschrieben.

Nachteilig an den Verfahren, die von Piperazin ausgehen, ist der Umstand, daß Piperazin kein gezielt hergestellter Stoff ist, sondern im allgemeinen als Nebenprodukt anderer Synthesen gebildet wird. Aus diesem Grund steht Piperazin nicht immer in gewünschtem Umfang und Preis zur Verfügung.

Eine Folge dieser Schwierigkeit ist die Entwicklung eines zweiten, allgemeinen Synthesekonzepts, das auf einer Ringschlußreaktion beruht.

So wird nach der Lehre der NL—A—7 605 640 N-Methyldiethanolamin mit Ammoniak und mit Hilfe wasserentziehender Mittel umgesetzt.

Nach dem Verfahren der JP—A—81/133 261 setzt man Diethanolamin mit Monomethylamin mit Hilfe von Heteropolysäuren um.

Nach dem Verfahren der US—A—2 525 223 werden N-Alkylpiperazine drucklos aus Diethanolamin bzw. dessen Derivaten und einem Methylamin bei erhöhter Temperatur in Gegenwart eines Katalysators hergestellt. Die EP—A—0 137 478 beschreibt ein Verfahren zur Herstellung von N-methylierten cyclischen Iminen aus Diolen und Monomethylamin in Druckbereichen von 1 bis 50 bar in Gegenwart kupferhaltiger Katalysatoren bei erhöhter Temperatur.

Die Verfahren des Standes der Technik gehen zwar teilweise von billigen Stoffen aus, weisen jedoch den Nachteil niedriger Ausbeuten aus. So wird in der NL—A—7 605 640 eine Selektivität von 13% angegeben. Damit ist die Synthese von N-Methylpiperazin völlig unwirtschaftlich.

In der Literatur sind auch noch andere Synthesewege zur Herstellung von N-Methylpiperazin beschrieben, die aber nur den Charakter von Laboratoriumsmethoden haben. Ein Beispiel ist die Umsetzung von N-Benzoylpiperazin mit einem Gemisch aus Ameisensäure und Formaldehyd und anschließende Verseifung, vgl. SU—A—0146314.

Der Erfindung liegt die Aufgabe zugrunde, ein Verfahren zur Herstellung von N-Methylpiperazin zur Verfügung zu stellen, das von billigen und jederzeit verfügbaren Stoffen ausgeht, sich ohne Schwierigkeiten in technische Größenordnungen übertragen läßt, wobei hohe Ausbeuten erzielt werden und die Nachteile der bisher bekannten Verfahren nicht aufweist.

Diese Aufgabe wird gelöst mit einem Verfahren zur Herstellung von N-Methylpiperazin aus Diethanolamin und Methylamin, das dadurch gekennzeichnet ist, daß man die Umsetzung bei Temperaturen von 180 bis 230°C, bei Drücken von 50 bis 300 bar und bei einem Molverhältnis Diethanolamin zu Monomethylamin von 1:5 bis 1:20 in Gegenwart von Wasserstoff an einem metallhaltigen Katalysator durchführt.

Nach einer bevorzugten Ausführungsform arbeitet man in im wesentlichen flüssiger Phase. Damit ist gemeint, daß im Reaktor eine flüssige Phase vorliegt, die beispielsweise von Gasblasen aus gasförmigem Wasserstoff durchsetzt sein kann. Die flüssige Phase kann kontinuierlich oder diskontinuierlich sein. Bevorzugt ist sie diskontinuierlich.

Der bevorzugte Temperaturbereich liegt zwischen 190 und 220°C. Bei niedriger Temperatur steigt der Anteil unverdampflicher Nebenprodukte, bei höherer Temperatur steigt der Anteil anderer Nebenprodukte. Piperazin erhält man stets in nur unwesentlichen Anteilen im Bereich um 1%. Das Molverhältnis Diethanolamin zu Monomethylamin liegt zwischen 1 zu 5 bis 1 zu 20. Mit fallendem Monomethylaminüberschuß steigt der Anteil unverdampflicher Nebenprodukte. Steigt der Monomethylaminüberschuß über den oberen Grenzbereich hinaus, wird der Anteil unverdampflicher Nebenprodukte kaum noch vermindert, stattdessen wrid die Wirtschaftlichkeit des Verfahrens zunehmend beeinträchtigt.

Der bevorzugte Druckbereich liegt zwischen 100 und 250 bar. Der über den Dampfdruck der beteiligten Stoffe hinausgehende Druck wird durch Wasserstoff aufgebracht. Die Anwesenheit von Wasserstoff ist der Selektivität und Aktivität des Katalysators förderlich.

Als metallhaltiger Katalysator kommt insbesondere ein metallhaltiger Trägerkatalysator in betracht. Der Ausdruck "metallhaltig" bedeutet, daß im Katalysator Metall oder Metallsalz (einschließlich Metalloxide) als aktive Komponente vorliegt. Als Beispiele für geeignete, metallhaltige Katalysatoren kann man nennen:

mit Kupfernitratlösung getränkte Strangpreßlinge aus aktiver Tonerde, die nach dem Trocknen und Calcinieren ca. 20 Gew.-% CuO enthalten,

mit einer Mischlösung der Nitrate von Kupfer, Nickel und Kobalt getränkte Strangpreßlinge aus aktiver Tonerde, die nach dem Trocknen und Calcinieren ca. 10 Gew.-% NiO, 10 Gew.-% CoO und 5 Gew.-% CuO enthalten,

durch gemeinsame Fällung aus einer Mischlösung hergestellte Partikel, die nach dem Trocknen und Calcinieren ca. 30 Gew.-% CuO, 10 Gew.-% NiO und 60 Gew.-% $Al_2O_3$ enthalten,

durch gemeinsame Fällung aus einer Mischlösung hergestellte Partikel, die nach dem Trocknen und Calcinieren ca. 45 Gew.-% CuO und 55 Gew.-% $Al_2O_3$ enthalten.

Derartige Katalysatoren sind beispielsweise beschrieben in DE—A—19 53 263, DE—A—24 45 303 und DE—A—31 25 662 beschrieben.

Besonders bevorzugt sind Katalysatoren, welche als aktive Komponente hauptsächlich Kupfer aufweisen. Derartige Katalysatoren können beispielsweise einen Gehalt von 10 bis 60% Kupfer in Form von metallischem Kupfer oder Kupferionen (einschließlich Oxiden) aufweisen. Als weitere Komponenten des Katalysators kommen Chrom, Mangan, Kobalt, Nickel, Silber in Betracht.

Besonders bevorzugt ist ein Trägerkatalysator, der etwa 35 bis 55 Gew.-% Kupfer, bezogen auf das Gesamtgewicht des Katalysators aufweist. Als Träger ist Aluminiumoxid, beispielsweise Gamma-Aluminiumoxid, besonders geeignet. Der Katalysator kann in unterschiedlicher Form vorliegen, beispielsweise in Form zylindrischer Tabletten oder in Form von Ringen und dergleichen. Besonders geeignete Abmessungen sind Höhe und Durchmesser von jeweils etwa 2 bis 7 mm.

Als Apparatur für die Durchführung der Reaktion und die Einstellung der erfindungsgemäßen Bedingungen sind die an sich bekannten Vorrichtungen wie z.B. Rohrreaktoren oder Schachtreaktoren geeignet. In einem solchen Reaktor ist der Katalysator in Form von Tabletten oder Strangpreßlingen fest angeordnet. Die bei den Reaktionsbedingungen überwiegend flüssige Zulaufmischung wird nach Vorwärmung auf die Reaktionstemperatur bevorzugt am Kopf des Reaktors eingebracht und durchströmt die Katalysatorschüttung in Form einer sogenannten Rieselphase von oben nach unten. Wegen der geringen Reaktionswärme erfolgt die Umsetzung nahezu isotherm. Bei Abkühlen des rohen Reaktionsproduktes löst sich darin eine geringe Menge Wasserstoff und das Methylamin. Beim Entspannen entweicht der gesamte gelöst mitgeführte Wasserstoff und ein großer Teil des Methylamins. Im Normalfall wird der Wasserstoff als Abgas abgeleitet, während das Methylamin zurückgewonnen und erneut zur Reaktion gebracht wird. In gleicher Weise wird das nach der Entspannung noch im Reaktionsgemisch enthaltene Methylamin behandelt.

Das Diethanolamin wird normalerweise schon bei einmaligem Durchgang durch den Reaktor vollständig umgesetzt. Neben dem Hauptprodukt entstehen in jeweils wenigen Prozentanteilen mehrere niedersiedende Stoffe, die im wesentlichen Spaltprodukte des Diethanolamins und deren Reaktionsprodukte mit Methylamin sind. Außerdem enthält das Reaktionsgemisch einige Prozente hochsiedende Stoffe, von denen einige nicht temperaturbeständig sind.

Zur Aufarbeitung des Reaktionsgemisches trennt man das unumgesetzte Methylamin ab und gewinnt es zurück. Das kann nach an sich bekannten Arbeitsweisen erfolgen. Auch die Abtrennung der in geringem Umfang entstehenden, hochsiedenden Nebenprodukte erfolgt auf übliche Weise. Besonders zweckmäßig erfolgt die endgültige Reinigung des N-Methylpiperazins durch eine Reindestillation. Auf diese Weise erhält man das Wertprodukt in Reinheiten von über 99,5% und in Ausbeuten, die bei Werten bis zu über 70% liegen.

Das erfindungsgemäße Verfahren kann kontinuierlich oder diskontinuierlich betrieben werden. Besonders zweckmäßig ist eine kontinuierliche Fahrweise.

Das nachstehende Beispiel dient zur weiteren Erläuterung der Erfindung.

Beispiel

In einem Reaktor mit 350 cm Länge und 4,3 cm Innendurchmesser waren 4 l Katalysator eingefüllt. Dieser bestand aus 45 Gew.-% Kupfer und 55 Gew.-% Aluminiumoxid und war zu zylindrischen Tabletten mit Höhe=Durchmesser=4 mm geformt. Eine Außenheizung sorgte für konstante Temperatur im Reaktor. Zum Reaktor gehörten zwei Vorratsgefäße für Diethanolamin (I) und für Monomethylamin (II), aus denen die vorgegebenen Mengen von 0,4 l/h (I) und 1,6 l/h (II) kontinuierlich zum Reaktor gepumpt wurden. Dem Reaktor war ein Vorheizer vorgeschaltet, in dem der Zulauf auf 200°C erwärmt wurde.

Der Druck im Reaktorsystem wurde mit Hilfe von hochgespanntem Wasserstoff auf 250 bar eingestellt.

Das Zulaufgemisch durchströmte die Katalysatorschicht in Form einer Rieselphase von oben nach unten. Nach dem Verlassen des Reaktors wurde auf 20°C gekühlt und das Produktgemisch in einem Hochdruck-Abscheider gesammelt. Von hier wurde in einer Stufe auf Atmosphärendruck entspannt, danach enthielt das Produktgemisch noch 15 Gew.-% Monomethylamin. Ohne Berücksichtigung des bei der Reaktion entstandenen Wassers und des gelösten Methylamins war das Produktgemisch zusammengesetzt aus: 71 Gew.-% Monomethylpiperazin, 1 Gew.-% Piperazin, 23 Gew.-% andere Nebenprodukte und 5 Gew.-% Abdampfrückstand.

Dieses Gemisch wurde durch eine zweistufige diskontinuierliche Destillation ausgearbeitet. In der ersten Stufe wurde zunächst das Methylamin und dann alles übrige vom unverdampflichen Rückstand abgetrennt. Diese Hauptfraktion wurde in der zweiten Stufe bei Atmosphärendruck fraktionierend destilliert. Bei einer Siedetemperatur von 137°C, gemessen am Kopf der Destillationskolonne, wurde das Methylpiperazin in einer Reinheit von 99,8% erhalten, bestimmt durch GC-Analyse, 4 m Carbowax.

**Patentansprüche**

1. Verfahren zur Herstellung von N-Methylpiperazin aus Diethanolamin und Methylamin, dadurch gekennzeichnet, daß man die Umsetzung bei Temperaturen von 180 bis 230°C, bei Drücken von 50 bis 300 bar und bei einem Molverhältnis Diethanolamin zu Monomethylamin von 1:5 bis 1:20 in Gegenwart von Wasserstoff an einem metallhaltigen Katalysator durchführt.

2. Verfahren nach Anspruch 1, dadurch gekennzeichnet, daß man in im wesentlichen flüssiger Phase arbeitet.

3. Verfahren nach einem der Ansprüche 1 oder 2, dadurch gekennzeichnet, daß man einen Kupfer enthaltenden Katalysator einsetzt.

**Revendications**

1. Procédé de préparation de N-méthylpipérazine à partir de diéthanolamine et de méthylamine, caractérisé en ce qu'on mène la réaction à des températures de 180 à 230°C, à des pressions de 50 à 300 bars et à un rapport molaire de la diéthanolamine à la monométhylamine de 1:5 à 1:20, en présence d'hydrogène sur un catalyseur contenant un métal.

2. Procédé selon la revendication 1, caractérisé en ce qu'on travaille en phase essentiellement liquide.

3. Procédé selon l'une des revendications 1 et 2, caractérisé en ce qu'on utilise un catalyseur contenant du cuivre.

**Claims**

1. A process for the preparation of N-methylpiperazine from diethanolamine and methylamine, wherein the reaction is carried out at from 180 to 230°C, at a pressure of from 50 to 300 bar and at a molar ratio of diethanolamine to monomethylamine of from 1:5 to 1:20 in the presence of hydrogen over a metal-containing catalyst.

2. A process as claimed in claim 1, wherein the operation is carried out in a substantially liquid phase.

3. A process as claimed in claim 1 or 2, wherein a copper-containng catalyst is employed.